(19) Europäisches Patentamt
European Patent Office
Office européen des brevets

(11) **EP 3 677 245 A1**

(12) **EUROPEAN PATENT APPLICATION**
published in accordance with Art. 153(4) EPC

(43) Date of publication:
**08.07.2020 Bulletin 2020/28**

(21) Application number: **18851745.2**

(22) Date of filing: **31.08.2018**

(51) Int Cl.:
***A61K 8/25*** *(2006.01)* ***A61K 8/06*** *(2006.01)*
***A61K 8/31*** *(2006.01)* ***A61K 8/89*** *(2006.01)*
***A61K 8/894*** *(2006.01)* ***A61K 8/895*** *(2006.01)*
***A61K 8/92*** *(2006.01)* ***A61Q 19/00*** *(2006.01)*

(86) International application number:
**PCT/JP2018/032410**

(87) International publication number:
**WO 2019/045056 (07.03.2019 Gazette 2019/10)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **01.09.2017 JP 2017168195**

(71) Applicant: **Shiseido Co., Ltd.**
**Tokyo 104-0061 (JP)**

(72) Inventors:
- **YABUSAKI, Yusuke**
  **Yokohama-shi**
  **Kanagawa 224-8558 (JP)**
- **SHIRAKAMI, Hirohito**
  **Yokohama-shi**
  **Kanagawa 224-8558 (JP)**
- **HIROSE, Yuka**
  **Yokohama-shi**
  **Kanagawa 224-8558 (JP)**
- **TAKAHASHI, Shigeo**
  **Yokohama-shi**
  **Kanagawa 224-8558 (JP)**

(74) Representative: **Ter Meer Steinmeister & Partner Patentanwälte mbB**
**Nymphenburger Straße 4**
**80335 München (DE)**

# (54) WATER-IN-OIL TYPE EMULSION COSMETIC

(57) The purpose of the present invention is to provide a solid or semisolid water-in-oil type emulsion cosmetic that has a hardness that can be said to be balm-like, the cosmetic having an excellent moisturizing effect, exhibiting upon use a unique sensation of blending as if melting when applied to skin, and being capable of stably incorporating a water-soluble or hydrophilic drug. The present invention pertains to a water-in-oil type emulsion cosmetic characterized by containing: (A) an organically modified clay mineral; (B) a surfactant having an HLB of no more than 6; (C) a wax; (D) a silicone elastomer; (E) a volatile oil; and (F) water.

[FIG. 1]

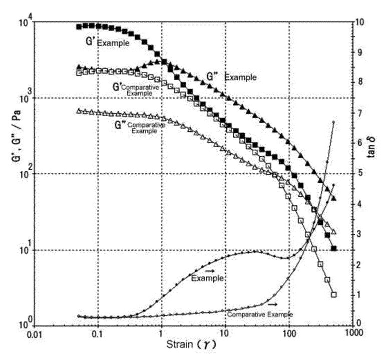


EP 3 677 245 A1

## Description

### Technical Field

The present invention relates to a water-in-oil type emulsified cosmetic. More specifically, the present invention relates to a water-in-oil type emulsified cosmetic that exhibits such a uniquely tactile sensation (texture) on use that while providing a sensation as if melting upon applying to the skin, despite a balm form having a hardness to some extent.

### Background Art

As a skin external preparation in the balm form, Vaseline®, Sonbahyu®, Shea Butter® from L'Occitane and the like are well known. These preparations comprise a highly viscous oily component, such as petrolatum, bahyu (horse fat), or shea butter, and exhibit an excellent moisturizing effect by covering the skin with an oily film. Particularly, it is known that a preparation containing shea butter provides the skin with a particular tactile sensation while melting on the skin. However, a problem is that such a preparation comprises a high viscous oily component, so that spreadability upon application can be low and stickiness can occur after application.

With regard to cosmetics, water-in-oil emulsified preparations in the form of solids or semisolids that comprise waxes, oily thickening agents, and the like in their external phases (oil phases) have been widely used mainly for makeup cosmetics such as lipsticks. Also for skin care cosmetics, Patent Document 1 proposes an oily solid cosmetic comprising a dextrin fatty acid ester, which has been conventionally used as an oily thickening agent, in combination with a naturally occurring solid fat and/or semisolid fat such as beeswax and shea butter, and an isoparaffin having a molecular weight and a kinematic viscosity in predetermined ranges, and Patent Document 1 states that the oily solid cosmetic is not sticky, exhibits a unique melting feeling and fits the skin well.

Patent Document 2 discloses an oily solid cosmetic comprising a dextrin fatty acid ester, a liquid paraffin and/or squalane, and triacyl glycerol, and states that the oily solid cosmetic has an excellent transparent appearance, an excellent feeling on use, and an excellent moisturizing feeling, and further excellent storage stability. However, Patent Documents 1 and 2 disclose oily cosmetics comprising no water, in which it is difficult to blend, for example, a water-soluble or hydrophilic active substance.

It was found that with regard to a water-in-oil type emulsified cosmetic comprising water in the internal phase thereof, the good stability, the good spreadability, and a dewy and fresh texture and a refreshing feeling can be obtained, when a lower alcohol, a volatile silicone oil, an organically modified clay mineral, a polyether-modified silicone surfactant (emulsifier) having an HLB of 7 or less, and a cooling agent are blended in the system comprising no solid or semisolid oil that can cause stickiness (Patent Document 3). However, the water-in-oil type emulsified cosmetic of Patent Document 3 comprises no wax, so that such a cosmetic does not have an enough hardness (viscosity) within the extent defined as a balm form and cannot cover the skin to exhibit a moisturizing effect.

### Citation List

### Patent Document

JP 2017-114780 A
JP 2017-114806 A
JP 3524717 B

## Summary of Invention

### Technical Problem

Therefore, an object of the present invention is to provide a solid or semisolid water-in-oil type emulsified cosmetic having a hardness to the extent that it can be said to be in the form of a balm, which has an excellent moisturizing effect and also exhibits such a unique tactile sensation as if melting and compatible with the skin when applied to the skin, and in which at the same time, a water-soluble or hydrophilic active ingredient can be stably blended.

### Solution to Problem

The present inventors have studied diligently in order to solve the above problems, and as a result found that

in a water-in-oil type emulsified cosmetic in which an organically modified clay mineral and a surfactant having an HLB of 6 or less, a wax, a silicone elastomer, and a volatile oil are blended, the organically modified clay mineral and the surfactant having an HLB of 6 or less can form an oil gel together with the oil to stabilize the emulsion, the wax can achieve the desired hardness, and the blending of the silicone elastomer and the volatile oil can suppress stickiness so that oiliness does not remain on the skin, leading to the completion of the present invention.
Specifically, the present invention provides a water-in-oil type emulsified cosmetic comprising:

(A) an organically modified clay mineral;
(B) a surfactant having an HLB of 6 or less;
(C) a wax;
(D) a silicone elastomer;
(E) a volatile oil; and
(F) water.

## Advantageous Effects of the Invention

The water-in-oil type emulsified cosmetic of the present invention is a solid or semisolid water-in-oil type emulsified cosmetic having a hardness to the extent that it can be called in the form of a balm, and has an excellent moisturizing effect by covering the skin with a film comprising a wax. In addition, such a unique tactile sensation that the cosmetic melts and deforms on the skin can be given in application, and a water-soluble or hydrophilic active ingredient can be stably blended. A cosmetic in the form of a balm exhibiting such a beauty effect has been unknown so far and is provided for the first time by the present invention.

## Brief Description of the Drawing

[FIG. 1] FIG. 1 is a graph showing the rheological properties of the water-in-oil type emulsified cosmetic of the present invention (Example 6) and Comparative Example 3 containing shea butter (L'Occitane's Shea Hand Cream (trade name)).

## Description of Embodiments

The water-in-oil type emulsified cosmetic (hereinafter also simply referred to as a "cosmetic") of the present invention will be described in detail below. The cosmetic of the present invention is in the form of a solid or a semisolid having a hardness to the extent that it can be called "in the form of a balm," as described above. The "in the form of a balm" and "solid or semisolid" herein mean exhibiting no fluidity at the time of storage at normal temperature (25°C). For example, the hardness at normal temperature (25°C) is preferably within the range of 5 to 200.
Here, the "hardness" herein is hardness ($\gamma$) represented by the following formula (1) as measured under the following conditions using a known measuring instrument such as a rheometer (manufactured by Fudo Kogyo):

$$\gamma = (G*L)/(l*a)(N/cm^2) \qquad (1)$$

wherein G: measured stress, L: sample thickness (mm), 1: compression distance (mm), a: cross-sectional area ($cm^2$) of needle.

(Measurement Conditions)

Needle diameter: 5.6 mm $\varphi$, penetration rate: 2 cm/min, penetration distance: 10 mm, measurement temperature: 25°C

### (A) Organically Modified Clay Mineral

The organically modified clay mineral used in the present invention is not limited as long as it is conventionally blendable into cosmetics and the like. For example, it is preferable to use a one obtainable by treating a clay mineral that is one kind of colloidal hydrous aluminum silicates having a three-layer structure and is represented by the following general formula with a quaternary ammonium salt type cationic surfactant.

$$(X, Y)_{2\text{-}3}(Si, Al)_4O_{10}(OPH)_2Z_{1/3}\cdot nH_2O$$

wherein X = Al, Fe(III), Mn(III), Cr(III), Y = Mg, Fe(II), Ni, Zn, Li, Z = K, Na, Ca.

Specifically, examples include a one obtained by treating a clay mineral, such as natural montmorillonite such as montmorillonite, saponite, and hectorite, synthetic montmorillonites (for example, one in which the OH groups in the above general formula are replaced by fluorine; commercial products include VEEGUM, KUNIPIA, and LAPONITE), or synthetic mica known by the name of sodium silicic mica or sodium or lithium taeniolite (commercial products include Dimonite: Topy Industries, Ltd.), with a quaternary ammonium salt type cationic surfactant.

As the quaternary ammonium salt type cationic surfactant used for organically modifying the clay mineral, one represented by the following general formula is preferred.

[Formula 1]

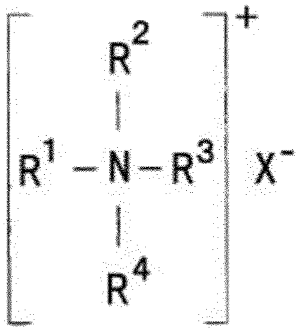

wherein $R_1$ represents an alkyl group having 10 to 22 carbon atoms or a benzyl group, $R_2$ represents a methyl group or an alkyl group having 10 to 22 carbon atoms, $R_3$ and R4 represent either an alkyl group or hydroxyalkyl group having 1 to 3 carbon atoms, and X represents a halogen atom or a methyl sulfate residue.

Specific examples include dodecyltrimethylammonium chloride, myristyltrimethylammonium chloride, cetyltrimethylammonium chloride, stearyltrimethylammonium chloride, arachyltrimethylammonium chloride, behenyltrimethylammonium chloride, myristyldimethylammonium chloride, cetyldimethylammonium chloride, stearyldimethylammonium chloride, arachyldimethylammonium chloride, behenyldimethylammonium chloride, cetyldiethylammonium chloride, stearyldiethylammonium chloride, arachyldiethylammonium chloride, behenyldiethylammonium chloride, benzyldimethylmyristylammonium chloride, benzyldimethylcetylammonium chloride, benzyldimethylstearylammonium chloride, benzyldimethylbehenylammonium chloride, benzyldimethylethylcetylammonium chloride, benzyldimethylethylstearylammonium chloride, distearyldimethylammonium chloride, dibehenyldihydroxyethylammonium chloride, and corresponding bromides, and further dipalmitylpropylethylammonium methyl sulfate. One or two or more of these can be arbitrarily selected.

When the clay mineral is treated with the above quaternary ammonium salt type cationic surfactant, the clay mineral can also be treated using a nonionic surfactant in combination. Examples thereof include ethylene oxide-added surfactants such as ether type surfactants such as oleyl ether to which 2 to 30 mol of polyoxyethylene is added (POE (2 to 30)), POE (2 to 35) stearyl ether, POE (2 to 20) lauryl ether, POE (1 to 20) alkyl phenyl ether, POE (6 to 18) behenyl ether, POE (5 to 25) 2-decylpentadecyl ether, POE (3 to 30) 2-decyltetradecyl ether, and POE (8 to 16) 2-octyldecyl ether, and ester type surfactants such as POE (4 to 60) hydrogenated castor oil, POE (3 to 14) fatty acid monoesters, POE (6 to 30) fatty acid diesters, and POE (5 to 20) sorbitan fatty acid esters, and further ether ester type surfactants such as POE (2 to 30) glyceryl monoisostearate, POE (10 to 60) glyceryl triisostearate, POE (7 to 50) hydrogenated castor oil monoisostearate, and POE (12 to 60) hydrogenated castor oil triisostearate, and polyhydric alcohol fatty acid ester type surfactants such as glycerin fatty acid esters such as decaglyceryl tetraoleate, hexaglyceryl triisostearate, diglyceryl diisostearate, and glyceryl monooleate. Among these, one or two or more fatty acid esters of diglycerin or higher polyglycerin, such as decaglyceryl tetraoleate, hexaglyceryl triisostearate, and diglyceryl diisostearate, or ethylene oxide-added nonionic surfactants such as POE-added ether type surfactants such as POE (2 to 12) lauryl ether, POE (6 to 15) behenyl ether, POE (5 to 20) 2-decylpentadecyl ether, POE (5 to 17) 2-decyltetradecyl ether, and POE (8 to 16) 2-octyldecyl ether, and POE-added ester type surfactants such as POE (10 to 20) hydrogenated castor oil, POE (5 to 14) oleic acid monoester, POE (6 to 20) oleic acid diester, and POE (5 to 10) sorbitan olein ester, and POE-added ether ester surfactants such as POE (3 to 15) glyceryl monoisostearate and POE (10 to 40) glyceryl triisostearate are preferably used.

The organically modified clay mineral used in the present invention can be obtained, for example, by subjecting

the above-described clay mineral and quaternary ammonium salt type cationic surfactant to dispersion and stirring treatment in a low boiling point solvent such as water, acetone, or a lower alcohol and then removing the low boiling point solvent.

The quaternary ammonium salt type cationic surfactant content in the organically modified clay mineral used in the present invention is preferably 60 to 140 milliequivalents (meq) based on 100 g of the clay mineral.

Typical examples of the organically modified clay mineral preferably used in the present invention include dimethylalkylammonium hectorite, benzyldimethylstearylammonium hectorite, and distearyldimethylammonium chloride-treated magnesium aluminum silicate. Commercial products include disteardimonium hectorite (BENTONE® 38VCG: manufactured by Elementis Specialties).

The amount of organically modified clay mineral is 0.1 to 0.5% by mass with respect to the total amount of the water-in-oil type emulsified cosmetic. The blending amount of organically modified clay mineral is preferably 0.5 to 3.0% by mass, more preferably 0.5 to 1.5% by mass. When the amount is less than 0.1% by mass, it is difficult to obtain a sufficient stability. When more than 5.0% by mass of the organically modified clay mineral is blended, it is hard to scrape (take) with fingers, and the spread on the skin becomes heavier, and so on, so that it is not preferred in terms of texture.

(B) Surfactant Having HLB of 6 or Less

The surfactant having an HLB of 6 or less used in the present invention is not particularly limited as long as it can be blended into a cosmetic and has an HLB value of 6 or less. Examples of the surfactant having an HLB of 6 or less include polyether-modified silicones, sorbitan fatty acid esters such as sorbitan tristearate, glycerin fatty acid esters such as glycerol monostearate and glycerol monooleate, and polyoxyethylene hydrogenated castor oil such as POE (5) hydrogenated castor oil and POE (7) hydrogenated castor oil. Among these, polyether-modified silicones are particularly preferably used.

For the polyether-modified silicones, specifically, those represented by the following formula are illustrated.

[Formula 2]

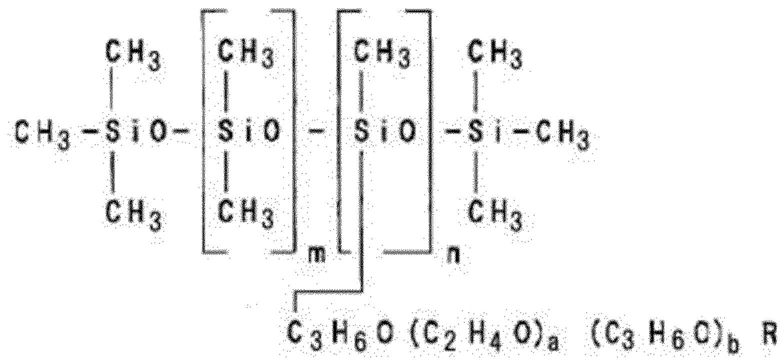

wherein R represents a hydrogen atom or an alkyl group having 1 to 5 carbon atoms, m represents a number of 1 to 150, n represents a number of 1 to 50, and a and b represent a number of 0 to 35.

The amount of the surfactant having an HLB of 6 or less is 0.1 to 5% by mass, preferably 0.5 to 3% by mass, and more preferably 1 to 2.5% by mass based on the total amount of the cosmetic.

The surfactant having an HLB of 6 or less contained in the water-in-oil type emulsified cosmetic of the present invention may be contained in a state of being adsorbed on the above component (A) organically modified clay mineral. In other words, in the present invention, it is possible to separately add the organically modified clay mineral (A) and the surfactant having an HLB of 6 or less (B) to produce the water-in-oil type emulsified cosmetic, or it is possible to previously treat the organically modified clay mineral (A) with the surfactant having an HLB of 6 or less (B) and blend the surfactant having an HLB of 6 or less in a state of being adsorbed on the organically modified clay mineral.

(C) Wax

The wax used in the present invention is not particularly limited as long as it is a wax that can be blended into a cosmetic. Specific examples include ceresin wax, paraffin wax, microcrystalline wax, carnauba wax, Japan wax, candelilla wax, rice bran wax, beeswax, shellac wax, and Chinese wax. Especially, one or a combination of two or more selected from ceresin, paraffin wax, and microcrystalline wax is preferably used.

The amount of the wax in the water-in-oil type emulsified cosmetic of the present invention is 1 to 15% by mass, preferably 3 to 9% by mass, and more preferably 3 to 5% by mass based on the total amount of the water-in-oil type emulsified cosmetic. When the content is more than 15% by mass, a good feel of use may be less likely to be obtained. When the content is less than 1% by mass, the stability over time may be impaired.

(D) Silicone Elastomer

The cosmetic of the present invention further contains (D) a silicone elastomer. By blending the silicone elastomer, such a unique feel that the cosmetic breaks, during application, can be provided, and stickiness after application can be suppressed.

Examples of the silicone elastomer used in the present invention include one or two or more powders of crosslinked silicone resins such as dimethicone crosspolymers, (dimethicone/phenyl divinyl methicone) crosspolymers, (dimethicone/vinyl dimethicone/methicone) crosspolymers, (dimethicone/vinyl dimethicone) crosspolymers, and (diphenyl dimethicone/vinyl diphenyl dimethicone/silsesquioxane) crosspolymers.

The silicone elastomer may be blended in a form of a silicone gel comprising a crosslinked silicone resin powder and a solvent. For such a silicone gel, commercial products can be used. Examples thereof can include KSG-15, KSG-18A, and KSG-16 (manufactured by Shin-Etsu Chemical Co., Ltd.).

The amount of the silicone elastomer in the cosmetic of the present invention is usually 0.5 to 5% by mass, preferably 0.5 to 4% by mass, more preferably 0.8 to 3% by mass, and particularly preferably 0.8 to 2% by mass. When the amount is less than 0.5% by mass, stickiness may be caused. When more than 5% by mass of the silicone elastomer is blended, it may be difficult to prepare a stable cosmetic.

(E) Volatile Oil

The volatile oil (E) used in the present invention is a (liquid) volatile oil having fluidity at normal temperature (25°C) and can be selected from oils that can be used in cosmetics and the like. Examples of the volatile oil (E) include low boiling point (boiling point at normal pressure: 260°C or less) isoparaffin-based hydrocarbon oils and silicone oils. One or two or more of them can be used.

Specific examples of the low boiling point isoparaffin-based hydrocarbon oils include isododecane, isohexadecane, and the like.

The low boiling point silicone oils include cyclic dimethylpolysiloxane having 4 to 6 silicon atoms and chain dimethylpolysiloxane having 2 to 5 silicon atoms. Specific examples can include cyclic silicone oils such as hexamethylcyclotrisiloxane (D3), octamethyltetracyclosiloxane (D4), decamethylcyclopentasiloxane (D5), and dodecamethylcyclohexasiloxane (D6), and linear or branched silicone oils having a viscosity of less than 5 cs (for example, dimethicone (viscosity: less than 5 cs)).

The amount of the volatile oil in the cosmetic of the present invention is 5 to 50% by mass, preferably 10 to 50% by mass, and more preferably 20 to 50% by mass. When the amount is less than 5% by mass, such a unique tactile sensation, as if the cosmetic melts and is compatible with the skin, is lost. When more than 50% by mass of the volatile fluid oil is blended, the emulsification stability decreases.

In addition, in the present invention, the ratio of the amount of the volatile oil (E) to the amount of the wax (C) ([(E)/(C)]) is preferably 50 or less. This ratio of the amount blended is further preferably 30 or less, particularly preferably 20 or less.

(F) Water

The cosmetic of the present invention is a water-in-oil type emulsified cosmetic containing (F) water in the internal phase. The cosmetic of the present invention contains water in the internal phase, and moreover a wax is blended into the external phase to maintain hardness, and therefore a water-soluble or hydrophilic drug or the like can be stably retained.

The amount of water blended in the cosmetic of the present invention is not particularly limited but is preferably 60% by mass or less. The amount of water blended is more preferably 50% by mass or less, further preferably 40% by mass or less.

In addition to the above essential components (A) to (F), the cosmetic of the present invention may contain other optional components that can be blended into the water-in-oil type emulsified cosmetic, as long as they do not impair the effects of the present invention. As the other optional components, the following can be illustrated, but the other optional components are not limited to these.

(G) Moisturizing Agent

By blending a moisturizing agent, the skin moisturizing effect of the cosmetic of the present invention can be further improved. Specific examples of the moisturizing agent include glycols such as propylene glycol, dipropylene glycol, 1,3-butylene glycol, 1,4-butylene glycol, diethylene glycol, triethylene glycol, and polyethylene glycol; glycerins such as glycerin, diglycerin, and polyglycerin; sugar alcohols such as sorbitol, mannitol, maltitol, xylitol, and erythritol; and saccharides such as fructose, glucose, galactose, maltose, lactose, and trehalose.
When the moisturizing agent is blended into the cosmetic of the present invention, the amount of the moisturizing agent blended is usually 1 to 15% by mass, preferably 2 to 12% by mass, and more preferably 3 to 10% by mass. When the amount blended is less than 1% by mass, the improvement of the moisturizing effect is not significant. When more than 15% by mass of the moisturizing agent is blended, stickiness may be caused conversely.

(H) Nonvolatile Fluid oil

By blending a nonvolatile fluid oil, the hardness of the cosmetic can be appropriately adjusted. The nonvolatile fluid oil used in the present invention is a (liquid) nonvolatile oil having fluidity at normal temperature (25°C) and can be selected from oils that can be used in cosmetics and the like. Examples of the nonvolatile fluid oil include hydrocarbon oils and/or silicone oils that are oily components having a boiling point of higher than 260°C at normal pressure. One or two or more of these can be used.
Specific examples of the nonvolatile fluid oil include linseed oil, camellia oil, macadamia nut oil, corn oil, olive oil, avocado oil, sasanqua oil, castor oil, safflower oil, apricot kernel oil, cinnamon oil, grape oil, almond oil, rapeseed oil, sesame oil, sunflower oil, wheat germ oil, rice germ oil, rice bran oil, cottonseed oil, soybean oil, peanut oil, tea seed oil, evening primrose oil, yolk oil, liver oil, triglycerin, glyceryl trioctanoate, and glyceryl triisopalmitate; ester oils such as octanoates such as cetyl octanoate, isooctanoates such as glyceryl tri-2-ethylhexanoate and pentaerythritol tetra-2-ethylhexanoate, laurates such as hexyl laurate, myristates such as isopropyl myristate and octyldodecyl myristate, palmitates such as octyl palmitate, stearates such as isocetyl stearate, isostearates such as isopropyl isostearate, isopalmitates such as octyl isopalmitate, oleates such as isodecyl oleate, adipic acid diesters such as diisopropyl adipate, sebacic acid diesters such as diethyl sebacate, ester oils such as diisostearyl malate; liquid hydrocarbon oils such as liquid paraffins and squalane; and further straight-chain silicones such as dimethylpolysiloxane, methylphenylpolysiloxane, and methylhydrogenpolysiloxane, amino-modified silicones, polyether-modified silicones, carboxy-modified silicones, alkyl-modified silicones, ammonium salt-modified silicones, and fluorine-modified silicones as silicone oils.
When the nonvolatile fluid oil is blended into the cosmetic of the present invention, the amount of the nonvolatile fluid oil is usually 30% by mass or less, preferably 20% by mass or less.

(I) Powder Component

The cosmetic of the present invention may contain various powder components that can be blended into skin cosmetics. Specific examples of the powder components include inorganic powders such as talc, kaolin, mica, silica, and zeolite; organic powders such as silicone resin powder, polyamide resin powders (nylon powders), polyethylene powders, polymethyl methacrylate powders, polystyrene powders, powders of copolymer resins of styrene and acrylic acid, and cellulose powders; inorganic white pigments such as titanium dioxide and zinc oxide; inorganic red pigments such as iron oxide (red oxide); inorganic yellow pigments such as yellow iron oxide and ocher; black pigments such as black iron oxide and carbon black; inorganic green pigments such as chromium oxide, chromium hydroxide, and cobalt titanate; inorganic blue pigments such as ultramarine blue and Prussian blue; pearl pigments such as titanium oxide-coated mica, colored titanium oxide-coated mica, bismuth oxychloride, and argentine; metal powder pigments such as aluminum powders and copper powders; organic pigments such as Red No. 201, Red No. 202, Red No. 204, Red No. 205, Red No. 220, Red No. 226, Red No. 228, Red No. 405, Orange No. 203, Orange No. 204, Yellow No. 205, Yellow No. 401, and Blue No. 404; organic pigments such as zirconium, barium, or aluminum lakes such as Red No. 3, Red No. 104, Red No. 106, Red No. 227, Red No. 230, Red No. 401, Red No. 505, Orange No. 205, Yellow No. 4, Yellow No. 5, Yellow No. 202, Yellow No. 203, Green No. 3, and Blue No. 1; and natural coloring matter such as chlorophyll and β-carotene. Especially, by blending a silicone resin powder, the usability improves further.
When the powder component is blended into the cosmetic of the present invention, the amount of the powder component is usually 1 to 10% by mass, preferably 2 to 5% by mass.

(J) Other Optional Components

Examples of optional components other than the above include thickening agents, ceramides, vitamins, ultraviolet absorbing agents, chelating agents, bactericides, preservatives, plant extracts, amino acids, various drugs, and

lower alcohols such as ethanol.

The cosmetic of the present invention can be produced according to a method conventionally used for a water-in-oil type emulsified cosmetic. To put it briefly, the cosmetic of the present invention can be produced by separately mixing oily components and aqueous components with heating as needed, emulsifying the aqueous phase in the oil phase, then filling a container with the obtained emulsion, and cooling the emulsion.

The cosmetic of the present invention exhibits such a unique feel of use that it breaks on the skin so as to melt, at the time of application, while being in the form of a balm. And the film spreading on the skin achieves an excellent moisturizing effect, and at the same time, the stably blended various drugs also exhibit an excellent beauty effect.

Therefore, the cosmetic of the present invention is particularly preferred for use as a beauty balm in which an active ingredient such as a whitening component is blended, or a foundation balm having an excellent moisturizing effect, and as a sun care preparation provided with an ultraviolet protection effect and SPF, and a hair care preparation that gives moderate moisture to the hair, and the cosmetic of the present invention can also be used as a fragrance balm whose fragrance can be enjoyed.

## Examples

The present invention will be described in more detail below by giving Examples, but the present invention is not limited in any way by these Examples. Amounts blended mean % by mass unless otherwise specified.

Water-in-oil type emulsified cosmetics were prepared according to formulations shown in the following Tables 1 to 4, and their properties were evaluated according to the following evaluation methods and evaluation criteria. The results are shown together in the tables.

"Emulsification Stability"

A test sample was allowed to stand at 50°C for 4 weeks and then returned to room temperature. The state was visually observed, and an evaluation was performed according to the following criteria:

A: No abnormality was observed.
B: Separation was slightly observed.
C: Separation was observed.

"Melting Sensation"

A test sample was applied to upper arms of 20 female panelists by hand. A survey was conducted for a melting sensation (= such a sensation that the test sample melts and is compatible (almost like absorbed)) during application, and an evaluation was performed according to the following criteria:

AA: 16 or more panelists answered that the test sample melted and was compatible.
A: 12 To 15 panelists answered that the test sample melted and was compatible.
B: 8 To 11 panelists answered that the test sample melted and was compatible.
C: 7 or less panelists answered that the test sample melted and was compatible.

[Table 1]

| | | Comparative Example 1 | Example 1 | Example 2 | Example 3 | Comparative Example 2 |
|---|---|---|---|---|---|---|
| Water | Ion-exchanged water | Remainder | Remainder | Remainder | Remainder | Remainder |
| Moisturizing agent | Glycerin | 2 | 2 | 2 | 2 | 2 |
| | Dipropylene glycol | 2 | 2 | 2 | 2 | 2 |
| | Polyethylene glycol 6000 | 1 | 1 | 1 | 1 | 1 |
| Organically modified clay mineral | Disteardimonium hectorite (BENTONE 38VCG) | 1 | 1 | 1 | 1 | 1 |

(continued)

| | | Comparative Example 1 | Example 1 | Example 2 | Example 3 | Comparative Example 2 |
|---|---|---|---|---|---|---|
| Wax | Paraffin wax | - | 0.92 | 2.76 | 4.6 | 2.76 |
| | Microcrystalline wax | - | 0.08 | 0.24 | 0.4 | 0.24 |
| Surfactant | PEG-9 polydimethyl siloxyphenyl trimethicone (polyether-modified silicone surfactant) | 2 | 2 | 2 | 2 | 2 |
| Volatile oil | Cyclomethicone | 48.2 | 48.2 | 48.2 | 48.2 | - |
| Nonvolatile oil | Dimethicone (viscosity: 5 cs or more) | - | - | - | - | 17.2 |
| | Diphenyl siloxyphenyl trimethicone | - | - | - | - | 7 |
| | Squalane | - | - | - | - | 20 |
| | Cetyl ethyl hexanoate | 3 | 3 | 3 | 3 | 3 |
| Elastomer | (Dimethicone/vinyl dimethicone) crosspolymer | 1.8 | 1.8 | 1.8 | 1.8 | 1.8 |
| Drug | Potassium 4-methoxysalicylate | 1 | 1 | 1 | 1 | - |
| Powder | Vinyl dimethicone/ methic one silsesquioxane crosspolymer | 3 | 3 | 3 | 3 | 3 |
| Stabilizer | | 0.5 | 0.5 | 0.5 | 0.5 | 0.5 |
| Preservative | Phenoxyethanol | 0.5 | 0.5 | 0.5 | 0.5 | 0.5 |
| Total | | 100 | 100 | 100 | 100 | 100 |
| Volatile oil/wax [(E)/(C)] | | - | 48.2 | 16.1 | 9.6 | 0 |
| Emulsion stability | | C | A | A | A | A |
| Texture (melting feeling) | | C | A | AA | A | C |

[Table 2]

| | | Example 4 | Example 5 | Example 6 | Example 7 | Example 8 |
|---|---|---|---|---|---|---|
| Water | Water | Remainder | Remainder | Remainder | Remainder | Remainder |
| Moisturizing agent | Glycerin | 3 | 2 | 2 | 2 | 2 |
| | Dipropylene glycol | 2 | 2 | 2 | 2 | 2 |
| | Polyethylene glycol 6000 | 1 | 1 | 1 | 1 | 1 |

(continued)

| | | Example 4 | Example 5 | Example 6 | Example 7 | Example 8 |
|---|---|---|---|---|---|---|
| Organically modified clay mineral | Disteardimonium hectorite (BENTONE 38VCG) | 1 | 1 | 1 | 1 | 1 |
| Wax | Ceresin wax | 5 | 5 | 3 | 5 | 3 |
| Surfactant | PEG-9 polydimethyl siloxyphenyl trimethicone (polyether-modified silicone surfactant) | 3 | 2 | 2 | 2 | 2 |
| Volatile oil | Cvclomethicone | 38.8 | 38.8 | 48.2 | 43.8 | 48.2 |
| Nonvolatile oil | Cetyl ethyl hexanoate | 5 | 5 | 3 | 5 | 3 |
| Elastomer | (Dimethicone/vinyl dimethicone) crosspolymer | 1.2 | 1.2 | 1.8 | 1.2 | 1.8 |
| Drug | Potassium 4-methoxysalicylate | - | - | - | - | 1 |
| Powder | Vinyl dimethicone/ methicone silsesquioxane crosspolymer | 4 | 5 | 3 | 5 | 3 |
| Stabilizer | Sodium chloride | 0.5 | 0.5 | 0.5 | 0.5 | 0.5 |
| Preservative | Phenoxyethanol | 0.5 | 0.5 | 0.5 | 0.5 | 0.5 |
| Total | | 100 | 100 | 100 | 100 | 100 |
| Volatile oil/wax [(E)/(C)] | | 7.8 | 7.8 | 16.1 | 8.8 | 16.1 |
| Emulsion stability | | A | A | A | A | A |
| Texture (melting feeling) | | A | A | AA | A | AA |

[Table 3]

| | | Example 9 | Example 10 | Example 11 | Example 12 | Example 13 |
|---|---|---|---|---|---|---|
| Water | Water | Remainder | Remainder | Remainder | Remainder | Remainder |
| Moisturizing agent | Glycerin | 2 | 2 | 2 | 2 | 2 |
| | Dipropylene glycol | 2 | 2 | 2 | 2 | 2 |
| | Polyethylene glycol 6000 | 1 | 1 | 1 | 1 | 1 |
| Organically modified clay mineral | Disteardimonium hectorite (BENTONE 38VCG) | 1 | 1 | 1 | 1 | 1 |

(continued)

| | | Example 9 | Example 10 | Example 11 | Example 12 | Example 13 |
|---|---|---|---|---|---|---|
| Wax | Paraffin wax | - | - | - | - | 0.92 |
| | Microcrystalline wax | 1 | - | - | 0.15 | 0.08 |
| | Candelilla wax | - | 1 | - | - | - |
| | Carnauba wax | - | - | 1 | - | - |
| | Polyethylene wax | - | - | - | 0.85 | - |
| Surfactant | PEG-9 polydimethyl siloxyphenyl trimethicone (polyether-modified silicone surfactant) | 2 | 2 | 2 | 2 | 2 |
| Volatile oil | Cvclomethicone | 48.2 | 48.2 | 48.2 | 48.2 | 55 |
| Nonvolatile oil | Cetyl ethyl hexanoate | 3 | 3 | 3 | 3 | 3 |
| Elastomer | (Dimethicone/vinyl dimethicone) crosspolymer | 1.8 | 1.8 | 1.8 | 1.8 | 1.8 |
| Drug | Potassium 4-methoxysalicylate | 1 | 1 | 1 | 1 | 1 |
| Powder | Vinyl dimethicone/ methicone silsesquioxane crosspolymer | 3 | 3 | 3 | 3 | 3 |
| Stabilizer | Sodium chloride | 0.5 | 0.5 | 0.5 | 0.5 | 0.5 |
| Preservative | Phenoxyethanol | 0.5 | 0.5 | 0.5 | 0.5 | 0.5 |
| Total | | 100 | 100 | 100 | 100 | 100 |
| Volatile oil/wax [(E)/(C)] | | 48.2 | 48.2 | 48.2 | 48.2 | 55 |
| Emulsion stability | | A | B | A | A | B |
| Texture (melting feeling) | | A | A | A | A | B |

[Table 4]

| | | Example 14 | Example 15 | Example 16 | Example 17 |
|---|---|---|---|---|---|
| Water | Ion-exchanged water | Remainder | Remainder | Remainder | Remainder |
| Moisturizing agent | Glycerin | 2 | 2 | 2 | 2 |
| | Dipropylene glycol | 4 | 4 | 2 | 2 |
| | Polyethylene glycol 6000 | 1 | 1 | 1 | 1 |
| Organically modified clay mineral | Disteardimonium hectorite (BENTONE 38VCG) | 1 | 1 | 1 | 1 |
| Wax | Paraffin wax | 2.76 | 2.76 | 4.14 | 3.68 |
| | Microcrystalline wax | 0.24 | 0.24 | 0.36 | 0.32 |

(continued)

| | | Example 14 | Example 15 | Example 16 | Example 17 |
|---|---|---|---|---|---|
| Surfactant | PEG-9 polydimethyl siloxyphenyl trimethicone (polyether-modified silicone surfactant) | 2 | 2 | 1 | 1 |
| | Polyglyceryl-2 diisostearate | - | - | 1.5 | 1.5 |
| Volatile oil | Cvclomethicone | - | - | 39 | 38 |
| | Dimethicone (viscosity: less than 5 cs) | 42 | 48.2 | - | - |
| Nonvolatile oil | Dimethicone (viscosity: 5 cs or more) | 6 | - | - | - |
| | Squalane | 3 | 3 | - | - |
| | Cetyl ethyl hexanoate | 3 | 3 | 3 | 3 |
| Elastomer | (Dimethicone/vinyl dimethicone) crosspolymer | 1.8 | - | - | - |
| | Polysilicone-11 | - | 1.8 | - | - |
| | Dimethicone crosspolymer | - | - | 0.9 | 1.6 |
| Drug | Betaine | 5 | 5 | - | - |
| Powder | Vinyl dimethicone/methicone silsesquioxane crosspolymer | 3 | 3 | 4.5 | - |
| | Methyl methacrylate crosspolymer | - | - | - | 5 |
| Stabilizer | Sodium chloride | 0.5 | 0.5 | 0.5 | 0.5 |
| Preservative | Phenoxyethanol | 0.5 | 0.5 | 0.5 | 0.5 |
| Total | | 100 | 100 | 100 | 100 |
| Volatile oil/wax [(E)/(C)] | | 14.0 | 16.1 | 8.7 | 9.5 |
| Emulsion stability | | A | A | A | A |
| Texture (melting feeling) | | AA | AA | AA | B |

As it is clear from the results shown in the above Tables 1 to 4, the emulsification stability is insufficient, and the unique feel when the cosmetic is applied to the skin is not obtained either, in Comparative Example 1, in which no wax is contained. In addition, stable emulsification is possible, but the unique feel of use is not obtained, in Comparative Example 2, in which no volatile oil is contained. In contrast to these, the cosmetics of the present invention (Examples 1 to 12 and 14 to 17) were excellent in both the emulsification stability and the feel of use. Slight decreases in properties were seen, though at practically unproblematic levels, in Example 13, in which the ratio of the amount of the volatile oil blended to the amount of the wax blended was more than 50.

Next, the rheological properties for the above Example 6 and a commercial product in which shea butter was blended (trade name: L'Occitane Shea Hand Cream) as Comparative Example 3 were measured.

The rheological measurement was performed at a temperature of 32°C at an angular frequency of 1 [1/s], using a rheometer (MCR301 manufactured by Anton Paar GmbH, 50 mm $\varphi$ cone plate), with the strain ($\gamma$) applied to the measurement sample increased from 0.05 to 500. The results are shown in Figure 1.

As shown in FIG. 1, along with the strain increases, the storage elastic modulus (G') and the loss elastic modulus (G") decrease, in both Example 6 according to the present invention and Comparative Example 3, that is a cream comprising shea butter. On the other hand, in the Example of the present invention, at the point of strain equal to about 1, tan $\delta$ (= G" (loss elastic modulus)/G' (storage elastic modulus)) is "1" and a change from elasticity superiority to viscosity superiority occurs, whereas in the Comparative Example, the point of tan $\delta$ = 1 shifts to the larger strain side. In other words, it is indicated that in the Comparative Example, the viscosity suddenly decreases while the cream is taking a time to be compatible with the skin, whereas in the Example of the present invention, such a feel that the cosmetic is gradually melting and becomes compatible with the skin is obtained.

Other formulation examples of the cosmetic according to the present invention are shown below. The invention of this application is not limited to these formulation examples.

Formulation Example 1: Whitening Balm

| Blended component | Amount blended (% by mass) |
|---|---|
| (1) purified water | the remainder |
| (2) glycerin | 2 |
| (3) dipropylene glycol | 2 |
| (4) polyethylene glycol 6000 | 1 |
| (5) disteardimonium hectorite (BENTONE 38VCG) | 1 |
| (6) microcrystalline wax | 0.2 |
| (7) paraffin wax | 1.8 |
| (8) PEG-9 polydimethyl siloxyphenyl trimethicone (polyether-modified silicone surfactant) | 2 |
| (9) cyclomethicone | 35 |
| (10) cetyl ethyl hexanoate | 3 |
| (11) (dimethicone/vinyl dimethicone) crosspolymer | 1.5 |
| (12) tranexamic acid | 2 |
| (13) vinyl dimethicone/methicone silsesquioxane crosspolymer | 3 |
| (14) sodium chloride | 0.5 |
| (15) phenoxyethanol | 0.5 |

Formulation Example 2: Beauty Balm

| Blended component | Amount blended (% by mass) |
|---|---|
| (1) purified water | the remainder |
| (2) glycerin | 5 |
| (3) dipropylene glycol | 5 |
| (4) polyethylene glycol 6000 | 1 |
| (5) disteardimonium hectorite (BENTONE 38VCG) | 1 |
| (6) microcrystalline wax | 0.3 |
| (7) paraffin wax | 2 |
| (8) PEG-9 polydimethyl siloxyphenyl trimethicone (polyether-modified silicone surfactant) | 2 |
| (9) cyclomethicone | 40 |
| (10) cetyl ethyl hexanoate | 3 |
| (11) (dimethicone/vinyl dimethicone) crosspolymer | 2 |
| (12) ascorbic acid glucoside | 2 |
| (13) vinyl dimethicone/methicone silsesquioxane crosspolymer | 3 |
| (14) sodium chloride | 0.5 |
| (15) phenoxyethanol | 0.5 |
| (16) citric acid | 0.02 |
| (17) sodium citrate | 0.18 |
| (18) potassium hydroxide | 0.4 |

## Claims

**1.** A water-in-oil emulsified cosmetic comprising:

(A) an organically modified clay mineral;
(B) a surfactant having an HLB of 6 or less;
(C) a wax;

(D) a silicone elastomer;
(E) a volatile oil; and
(F) water.

**2.** The cosmetic according to claim 1, wherein a ratio of an amount of said volatile oil (E) to an amount of said wax (C), ([(E)/(C)]), is at most 50.

**3.** The cosmetic according to claim 1 or 2, further comprising:
at least a powder component selected from the group consisting of a silicone resin powder and a polymethyl methacrylate powder.

**4.** The cosmetic according to any one of claims 1 to 3, wherein said cosmetic is provided in at least a form selected from a group consisting of a solid form having a hardness of 5 to 200 and a semisolid form having a hardness of 5 to 200.

[FIG. 1]

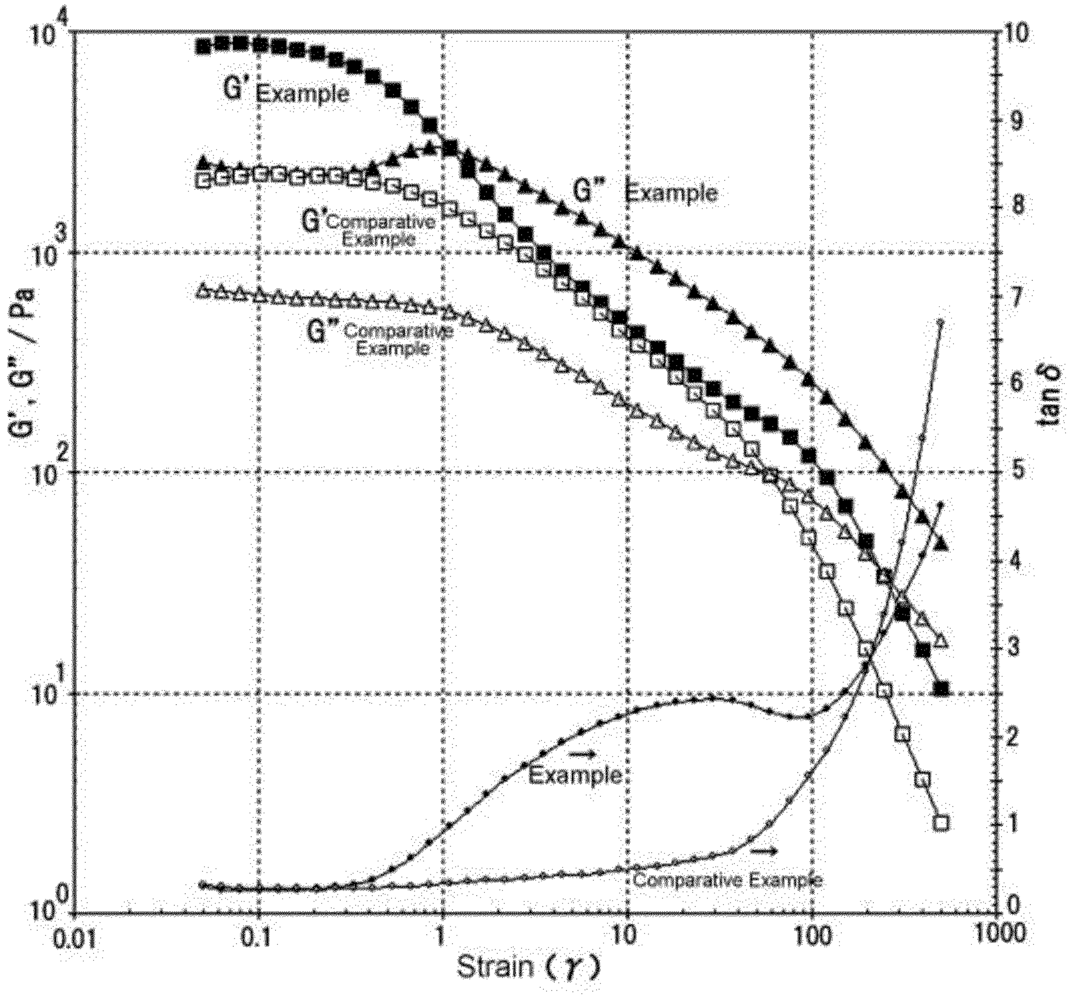
G' Example
G" Example
G' Comparative Example
G" Comparative Example
Example
Comparative Example
G', G" / Pa
tan δ
Strain (γ)
$10^4$
$10^3$
$10^2$
$10^1$
$10^0$
0.01
0.1
1
10
100
1000
10
9
8
7
6
5
4
3
2
1
0

## INTERNATIONAL SEARCH REPORT

International application No.
PCT/JP2018/032410

A. CLASSIFICATION OF SUBJECT MATTER
Int.Cl. A61K8/25(2006.01)i, A61K8/06(2006.01)i, A61K8/31(2006.01)i, A61K8/89(2006.01)i, A61K8/894(2006.01)i, A61K8/895(2006.01)i, A61K8/92(2006.01)i, A61Q19/00(2006.01)i

According to International Patent Classification (IPC) or to both national classification and IPC

B. FIELDS SEARCHED

Minimum documentation searched (classification system followed by classification symbols)
Int.Cl. A61K8/00-8/99, A61Q1/00-90/00

Documentation searched other than minimum documentation to the extent that such documents are included in the fields searched

| | |
|---|---|
| Published examined utility model applications of Japan | 1922-1996 |
| Published unexamined utility model applications of Japan | 1971-2018 |
| Registered utility model specifications of Japan | 1996-2018 |
| Published registered utility model applications of Japan | 1994-2018 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| Y | JP 2010-500400 A (THE PROCTER & GAMBLE COMPANY) 07 January 2010, claims 1-2, 7, paragraphs [0005], [0009], [0039]-[0043], [0053]-[0055] & US 2008/0038216 A1, claims 1, 3-5, 14, paragraphs [0005], [0010], [0040]-[0044], [0054]-[0056] & WO 2008/018045 A2 & CA 2658617 A1 & KR 10-2009-0023728 A & CN 101500527 A | 1, 3-4 |
| Y | WO 2015/174241 A1 (SHISEIDO CO., LTD.) 19 November 2015, claims 1-3, paragraphs [0012]-[0016], [0021]-[0027], [0036], [0043]-[0055], tables 1-5, paragraphs [0060]-[0061], treatment example 3 & US 2017/0266082 A1, claims 4-7, paragraphs [0018]-[0026], [0043]-[0049], [0060], [0069]-[0097], tables 1-5, paragraphs [0103]-[0104], treatment example 3 & JP 2015-218113 A & EP 3143984 A1 & KR 10-2016-0139042 A & CN 106456510 A | 1-4 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 22 November 2018 (22.11.2018) | 04 December 2018 (04.12.2018) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2018/032410

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2017-31149 A (SHISEIDO CO., LTD.) 09 February 2017, claim 1, paragraphs [0010], [0014] & US 2017/0056302 A1, claim 1, paragraphs [0015]-[0016], [0028] & WO 2016/017188 A2 & CN 106561081 A & KR 10-2018-0033433 A | 1-4 |
| Y | JP 2009-137900 A (SHISEIDO CO., LTD.) 25 June 2009, claim 1, paragraphs [0008], [0022]-[0023] (Family: none) | 1-4 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2017114780 A
- JP 2017114806 A
- JP 3524717 B